# EUROPEAN PATENT APPLICATION

(11) **EP 2 482 095 A2**
(43) Date of publication of application: **01.08.2012**
(21) Application number: 12152380.7
(22) Date of filing: 25.01.2012
(51) Int. Cl.: G01S 7/52

(54) **Receiving circuit for an ultrasonic imaging probe**

(30) Priority: 28.01.2011 US 201113016783
(71) Applicant: General Electric Company, Schenectady, NY 12345 (US)
(72) Inventor: Amamiya, Shinichi, Tokyo, 191-8503 (JP); Haider, Bruno, Niskayuna, NY New York 12309 (US); Rao, Naresh Kesavan, Niskayuna, NY New York 12309 (US)
(74) Representative: Bedford, Grant Richard

(57) **Abstract**

A receiving circuit 1 in an ultrasonic probe 102 that includes an ultrasonic transducer configured to receive ultrasonic waves is provided. The receiving unit includes an amplification unit 2 configured to amplify an echo signal received at the ultrasonic transducer. The amplification unit 2 includes a current output amplifier. The receiving circuit further includes a delay unit 3 configured to provide a delay time to output signals of the amplification unit 2.

## Description

The present invention relates generally to a receiving circuit including an amplification unit that amplifies ultrasonic echo signals and a delay unit that provides output signals of the amplification unit with a predetermined delay time and an ultrasonic probe and an ultrasonic image displaying apparatus equipped with this receiving circuit.

In ultrasonic image displaying apparatuses, an ultrasonic wave is transmitted from multiple ultrasonic transducers provided in an ultrasonic probe and an echo signal is received at each ultrasonic transducer. An echo signal received at each ultrasonic transducer is inputted to a receiving circuit and phased and added there. As a result, one reception beam is formed.

At the receiving circuit, an echo signal is amplified at an amplification unit provided in each ultrasonic transducer. To output signals of each amplification unit, a predetermined delay at a delay unit is given, and addition is performed at an adding unit. (Refer to Japanese Unexamined Patent Publication No. 2010-68957, for example.)

Each above amplification unit conventionally includes a voltage amplifier that outputs voltage. When a voltage amplifier is used, however, a buffer amplifier is required in the stage subsequent to the voltage amplifier and heat due to energy loss according thereto is produced.

When a voltage amplifier is used, an adder for adding output signals is required in addition to the above-mentioned buffer amplifier. In addition, since the frequency characteristic of the voltage amplifier is of wideband and thus a low-pass filter is required. This limits downsizing of each receiving circuit.

In general, the above-mentioned receiving circuits are provided in the main unit of an ultrasonic image displaying apparatus. The present inventors considered providing the above-mentioned receiving circuit in an ultrasonic probe. As mentioned above, however, downsizing of conventional receiving circuits is limited and it is difficult to provide them in an ultrasonic probe without change. In ultrasonic probes that are gripped by an operator, the production of heat poses a problem as compared with cases where a receiving circuit is provided in the main unit of an apparatus.

A first aspect of the invention includes a receiving circuit having an amplification unit that amplifies echo signals received at an ultrasonic transducer that receives ultrasonic waves and a delay unit that provides output signals of the amplification unit with a delay time. The receiving circuit is provided in an ultrasonic probe including the ultrasonic transducer and the amplification unit is comprised of a current output amplifier.

A second aspect of the invention is a receiving circuit according to the first aspect of the invention in which the delay unit includes: a capacitor onto which the output current of the current output amplifier is integrated; a write switch for writing the output current to this capacitor; and a read switch for reading charge from the capacitor.

A third aspect of the invention is a receiving circuit according to the second aspect of the invention in which the capacitor is charged with the output current while the write switch is on.

A fourth aspect of the invention is a receiving circuit according to the second aspect of the invention in which the following is implemented: the delay unit includes multiple capacitors and multiple write switches and read switches mentioned above; and the capacitors, write switches, and read switches form parallel circuits.

A fifth aspect of the invention is a receiving circuit according to the fourth aspect of the invention in which the following is implemented: when any one of the write switches is turned on, the others are turned off; and the current output amplifier is connected to any of the capacitors through the write switch in on state.

A sixth aspect of the invention is a receiving circuit according to the second aspect of the invention in which the on time of the write switches and/or the read switches is adjustable.

A seventh aspect of the invention is a receiving circuit according to the second aspect of the invention in which the delay time is a time from when each the write switch is turned off to when the corresponding read switch is turned on.

An eighth aspect of the invention is a receiving circuit according to the second aspect of the invention in which the capacitance of the capacitor is smaller than the following capacitance: the capacitance of a cable that connects the ultrasonic probe and the main unit of the ultrasonic image displaying apparatus.

A ninth aspect of the invention is a receiving circuit according to the second aspect of the invention in which an active charge amplifier circuit is provided in the stage subsequent to the read switch.

A tenth aspect of the invention is a receiving circuit according to the second aspect of the invention in which the following is implemented: one end of the write switch is connected with the current output amplifier and the other end thereof is connected with one end of the capacitor; the other end of the capacitor is connected with ground; and one end of the read switch is connected with the one end of the capacitor and the other end thereof is connected with an output line.

An eleventh aspect of the invention is a receiving circuit according to the second aspect of the invention in which the following is implemented: the write switch includes a first switch and a second switch that are synchronously turned on and off; the read switch includes a third switch and a fourth switch that are synchronously turned on and off; one end of the first switch is connected with an output-side first terminal of the current output amplifier and the other end thereof is connected with one end of the capacitor; one end of the second switch is connected with the other end of the capacitor and the other end thereof is connected with an output-side second terminal of the current output amplifier; one end of the third switch is connected with the one end of the capacitor and the other end thereof is connected with the output line; and one end of the fourth switch is connected with the other end of the capacitor and the other end thereof is connected with ground.

A twelfth aspect of the invention is a receiving circuit according to the first aspect of the invention in which in an output line from the delay unit, output currents of the delay unit are added.

A thirteenth aspect of the invention is a receiving circuit according to the first aspect of the invention in which the delay unit is provided for each of the ultrasonic transducers.

A fourteenth aspect of the invention is a receiving circuit according to the first aspect of the invention in which the delay unit is provided in common to ultrasonic transducers in multiple channels.

A fifteenth aspect of the invention is a receiving circuit according to the fourteenth aspect of the invention in which the delay unit is singularly provided in common to ultrasonic transducers in all the channels.

A sixteenth aspect of the invention is a receiving circuit according to the fourteenth aspect of the invention in which multiple delay units mentioned above are provided in common to ultrasonic transducers in some of all the channels.

A seventeenth aspect of the invention is a receiving circuit according to the fourteenth aspect of the invention in which output currents from the amplification units are added in the delay unit or in an output line from the delay unit.

An eighteenth aspect of the invention is a receiving circuit according to the first aspect of the invention in which the current output amplifier is either of the following: a V/I amplifier that amplifies input signals that are voltage signals and converts them into current signals and outputs the current signals; and an I/I amplifier that amplifies input signals that are current signals and outputs current signals.

A nineteenth aspect of the invention is an ultrasonic probe provided with a receiving circuit according to the first aspect of the invention.

A twentieth aspect of the invention is an ultrasonic image displaying apparatus equipped with an ultrasonic probe according to the nineteenth aspect of the invention.

According to various of the above aspects of the invention, each amplification unit of the receiving circuit may include a current output amplifier. Without an adder, therefore, currents outputted from the amplification units in the individual channels are added at an output line in the stage subsequent to the amplification units. This obviates necessity for an adder. The frequency characteristic of each the current output amplifier is such that the gain is reduced as the frequency is increased toward the center frequency of ultrasonic waves. This obviates necessity for a low-pass filter or even a simple low-pass filter is sufficient. Since each the amplification unit outputs current, it is unnecessary to provide a buffer amplifier on the subsequent stage side. Because of the foregoing, it is possible to reduce the size of a receiving circuit and suppress the production of heat more than conventional. As a result, the receiving circuit can be provided in an ultrasonic probe.

Various aspects and embodiments of the present invention will now be described in connection with the accompanying drawings, in which:
FIG. 1 is a schematic diagram illustrating an example of the embodiments of an ultrasonic image displaying apparatus;
FIG. 2 is a block diagram illustrating a receiving circuit in a first embodiment;
FIG. 3 is a diagram illustrating the configuration of a delay unit in the receiving circuit illustrated in FIG. 2;
FIG. 4 is a diagram explaining the timing with which the write switches and read switches in the delay unit illustrated in FIG. 3 are turned on and off;
FIG. 5 is a block diagram illustrating a receiving circuit including an active current output circuit;
FIG. 6 is a diagram illustrating an example of an active current output circuit;
FIG. 7 is a graph indicating the frequency characteristic of a current output amplifier;
FIG. 8 is an explanatory diagram illustrating a case where delay times are adjusted by varying times for which a write switch and a read switch are on;
FIG. 9 is a diagram illustrating the configuration of a delay unit in a second modification;
FIG. 10 is a diagram illustrating a delay unit in which a first switch and a second switch of a write circuit are synchronously on;
FIG. 11 is a diagram illustrating a delay unit in which a first switch and a second switch of another write circuit are synchronously on;
FIG. 12 is a diagram illustrating a delay unit in which a first switch and a second switch of another write circuit are synchronously on;
FIG. 13 is a diagram illustrating a delay unit in which a third switch and a fourth switch of a read circuit are synchronously on;
FIG. 14 is a diagram illustrating a delay unit in which a third switch and a fourth switch of another read circuit are synchronously on;
FIG. 15 is a diagram illustrating a delay unit in which a third switch and a fourth switch of another read circuit are synchronously on;
FIG. 16 is a block diagram illustrating a receiving circuit in a second embodiment;
FIG. 17 is a diagram illustrating the configuration of the delay unit in the receiving circuit illustrated in FIG. 16;
FIG. 18 is a block diagram illustrating a receiving circuit in a third embodiment; and
FIG. 19 is a diagram illustrating the configuration of a delay unit in the receiving circuit illustrated in FIG. 18.

Hereafter, detailed description will be given to embodiments of the invention with reference to the diagrams.

### First Embodiment

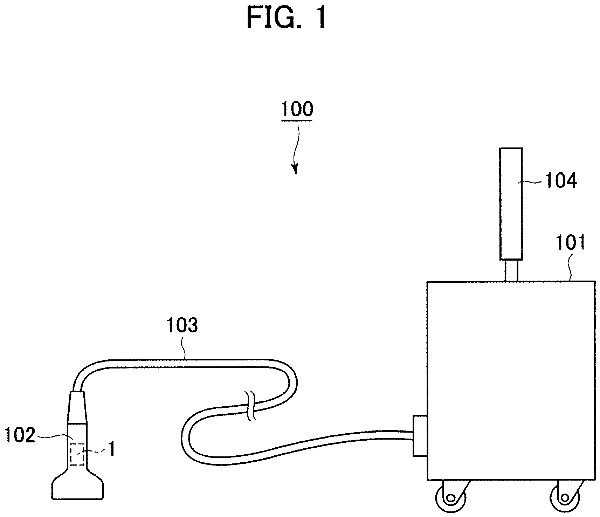

Description will be given to a first embodiment with reference to FIGS. 1-7. As illustrated in FIG. 1, an ultrasonic image displaying apparatus 100 includes an apparatus main unit 101 and an ultrasonic probe 102 connected with the apparatus main unit 101. The ultrasonic probe 102 is connected with the apparatus main unit 101 through a cable 103.

The ultrasonic probe 102 is provided with a receiving circuit 1 to which ultrasonic echo signals received at ultrasonic transducers are inputted. The ultrasonic probe 102 may be provided with a transmitting circuit though not especially shown in the diagram. The transmitting circuit drives the ultrasonic transducers of the ultrasonic probe 102 under a predetermined transmission condition and scans a scan surface with an ultrasonic beam in a sound-ray sequential manner.

The apparatus main unit 101 has output signals from the receiving circuit 1 inputted thereto through the cable 103 and the signals are A-D converted at an A-D conversion unit, not shown. Based on echo signals obtained after the A-D conversion, an ultrasonic image is generated and this ultrasonic image is displayed in the display 104 of the apparatus main unit 101.

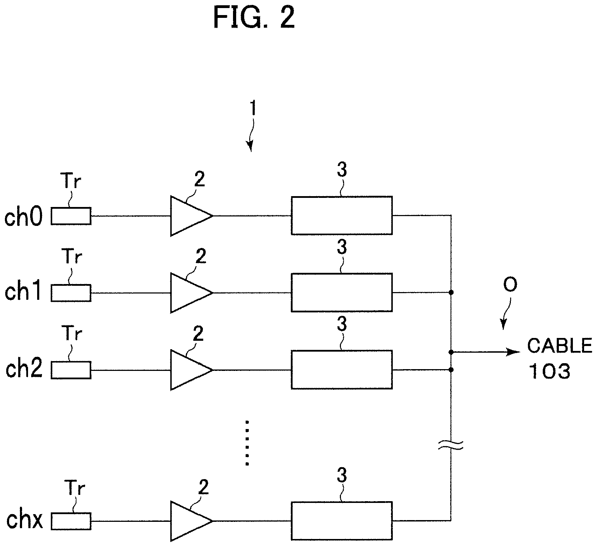

Detailed description will be given to the receiving circuit 1 with reference to FIG. 2 and the following diagrams. The receiving circuit 1 includes an amplification unit 2 and a delay unit 3. The amplification unit 2 and the delay unit 3 are provided for each of multiple ultrasonic transducers Tr in channel 0 up to channel x (x is an arbitrary natural number) provided in the ultrasonic probe 102. An echo signal received at each ultrasonic transducer Tr is amplified at an amplification unit 2 and then receives a predetermined delay time at a delay unit 3.

The amplification unit 2 includes a current output amplifier. This current output amplifier is either of the following: a V/I amplifier that amplifies input signals that are voltage signals and coverts them into current signals and outputs the current signals; and an I/I amplifier that amplifies input signals that are current signals and outputs current signals.

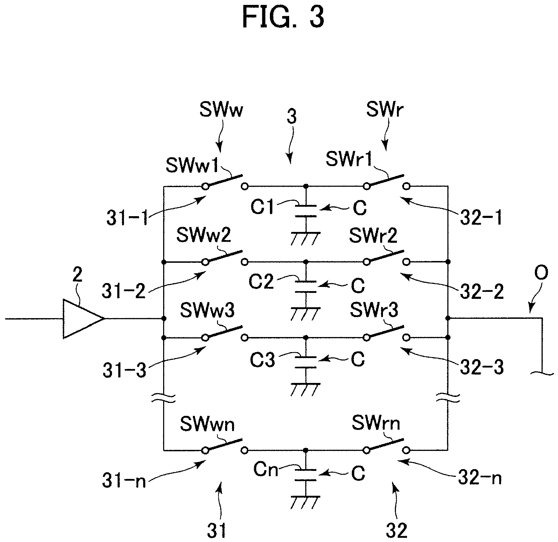

As illustrated in FIG. 3, each delay unit 3 includes capacitors C, write switches SWw, and read switches SWr. Incidentally, FIG. 3 shows a delay unit 3 and an amplification unit 2 equivalent to one channel. There are provided multiple capacitors C, multiple write switches SWw, and multiple read switches SWr. That is, there are provided capacitors C1, C2, C3, ..., Cn (n is a natural number), write switches SWw1, SWw2, SWw3, ..., SWwn, and read switches SWr1, SWr2, SWr3, ..., SWrn. The individual capacitors C, write switches SWw, and read switches SWr are connected in parallel to one another. Current integration is carried out by this parallel circuit.

One end of each of the write switches SWw is connected with an amplification unit 2 and the other end thereof is connected with one end of a capacitor C. The other end of the capacitor C is connected with ground. One end of each of the read switches SWr is connected with one end of a capacitor and the other end thereof is connected with an output line O.

Each write switch SWw, each capacitor C, and ground form a write circuit 31 that writes the output current of the amplification unit 2 to the capacitor C. As the write circuit 31, multiple write circuits 31-1, 31-2, 31-3, ..., 31-n are provided in parallel. In each write circuit 31, the output current of the amplification unit 2 is integrated in the capacitor C when the write switch SWw is on.

Each read switch SWr, each capacitor C, and ground form a read circuit 32 that reads the charge previously integrated onto the capacitor C. As the read circuit 32, multiple read circuits 32-1, 32-2, 32-3, ..., 32-n are provided in parallel. In each read circuit 32, the charge integrated onto the capacitor C is read when the read switch SWr is on.

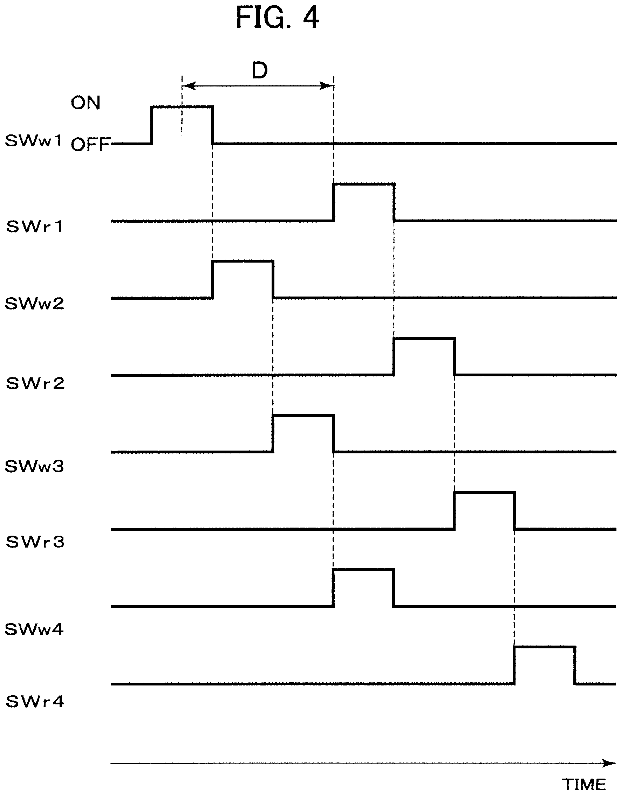

Description will be given to the timing with which the write switches SWw and the read switches SWr are turned on and off. As illustrated in FIG. 4, when any one of the write switches SWw is turned on, the others are turned off. As a result, at any given time the amplification unit 2 is only connected with one capacitor C through the write switch SWw in on state.

Similarly, when any one of the read switches SWr is turned on, the others are turned off.

The write switches SWw and the read switches SWr are sequentially turned on. More specific description will be given. The write switch SWwm (m is a natural number of 2 to n) is turned on when the previous write switch SWw(m-1) transitions from on to off. For example, when the write switch SWw1 transitions from on to off, the write switch SWw2 transitions from off to on; and when the write switch SWw2 transitions from on to off, the write switch SWw3 is turned on. As a result, in each channel, the output current from the amplification unit 2 is sequentially written to the individual capacitors C. Similarly, the read switch SWrm (m is a natural number of 2 to n) is turned on when the previous read switch SWr(m-1) transitions from on to off.

All the write switches SWw1 to SWwn are turned on for equal length but nonoverlapping time periods. All the read switches SWr1 to SWrn are turned on for equal length but nonoverlapping time periods.

Incidentally, there may be provided a circuit for discharging the current remaining in a capacitor C after the current in the capacitor C is read by a read switch SWr.

The delay time D provided at each delay unit 3 is the time from the center of the integration period (where write switch SWw is on) to when the read switch SWr transitions from off to on. The delay time D may differ from channel to channel.

The output line O is a low impedance node through which the charge read from each capacitor C is transferred and the output lines O from the delay units 3 in the individual channels may combine to form a charge summing node. (Refer to FIG. 2.) Therefore, the charge read from the capacitor C by the read switch SWr in some channel is added to the charges read from the capacitors C in the other channels at the output line O. This output line O continues to the cable 103 and the summed charge is inputted to the apparatus main unit 101 through the cable 103.

It is desirable that the capacitance of each of the capacitors C is smaller than the capacitance of the cable 103 so that the current integrated onto the capacitor C is efficiently transferred to the output line O and the cable 103. Therefore, it is desirable to select a cable whose capacitance is larger than the capacitance of each of the capacitors C.

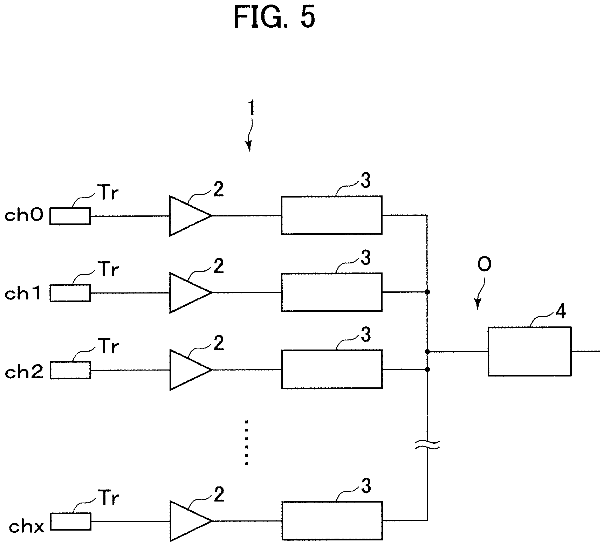

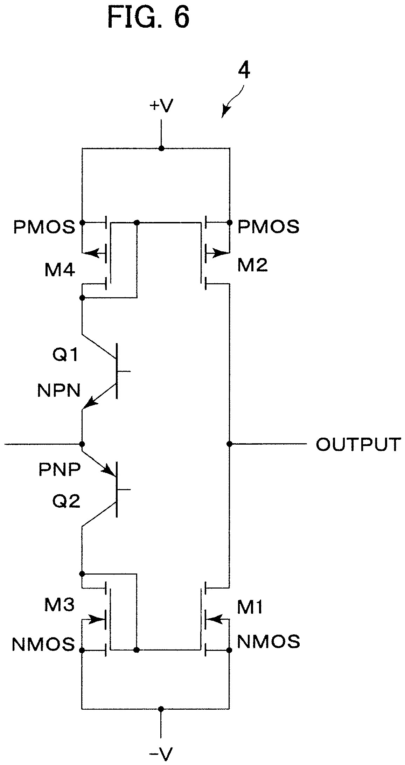

However, when the cable capacitance is not larger than the capacitance of the integration / delay capacitor C, the following measure may be taken so that the charge of the capacitor C can be transferred to the output line O. As illustrated in FIG. 5, an active current output circuit 4 is provided at the output line O. This circuit presents a low impedance to the output line O and thus facilitates an efficient transfer of the charge on capacitor C. For this active charge amplifier circuit 4, for example, one configured as illustrated in FIG. 6 can be adopted.

When a cable whose capacitance is larger than the capacitance of each capacitor C is not used as the cable 103, an additional capacitor may be provided at the output line O (diagram omitted) instead of providing the active current output circuit. In this case, the charge of the capacitor C can be transferred to the output line O by temporarily storing the charge on the additional capacitor.

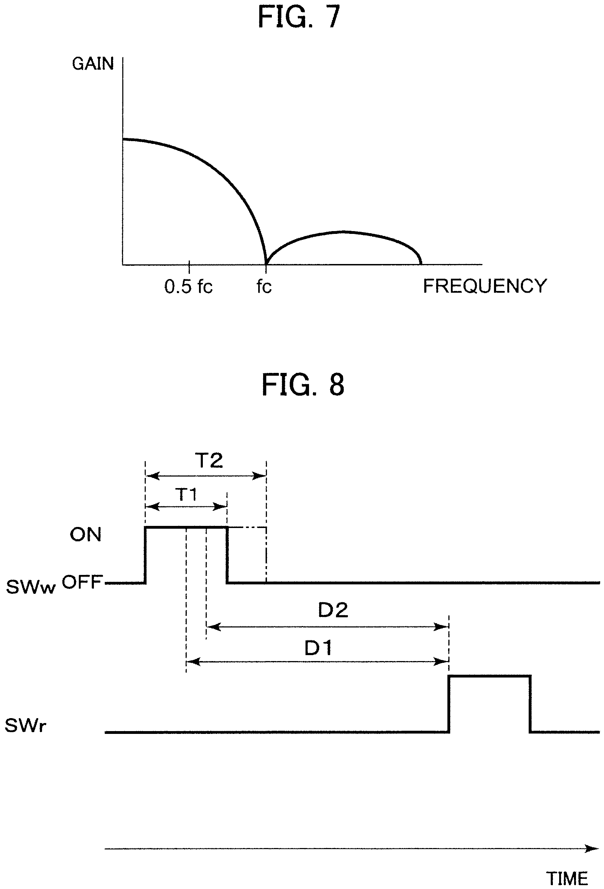

According to the example embodiment described up to this point, each amplification unit 2 includes a current output amplifier, specifically, a V/I amplifier or an I/I amplifier. Therefore, a current is outputted from the amplification unit 2 in each channel and these currents are integrated in the delay capacitors C and added at the output line O without provision of an adder. This obviates the necessity for an adder. Since currents are integrated, the frequency characteristic of each the current output amplifier is of the SINC function as illustrated in FIG. 7. In this function, the gain is reduced for higher frequencies. This makes it unnecessary to provide a low-pass filter in the stage subsequent to the amplification unit 2 or makes it possible to simplify the low-pass filter. Since the output of the amplification unit 2 is a current, it is unnecessary to provide a buffer amplifier on the subsequent stage. Because of the foregoing, it is possible to reduce the size of the receiving circuit 1 and reduce the power consumption compared to conventional voltage sampling. Therefore, the receiving circuit 1 can be provided in the ultrasonic probe 102.

It is said that sampling methods using current integration are susceptible to clock jitter and are not conducive to obtaining a sufficient S/N. However, in the preferred embodiment, each amplification unit 2 is always connected to one of the capacitors C and is constantly charging one of the capacitors C. This results in a first order cancellation of clock jitter induced noise. For example if the clock that turns off SWw(m) is slightly delayed then capacitor m will integrate too much charge. However, this clock delay will cause the next capacitor (m+1) to integrate less charge by exactly the amount of the excess charge on capacitor m. When the charges are being read out by the read switches, the total charge is unchanged by the clock jitter; the effect of the clock jitter is merely a delay of the excess charge. According to this example, therefore, it is possible to make the influence of clock jitters equal to that in voltage sampling using a voltage amplifier as in conventional cases.

Description will be given to modifications to the first embodiment. A first modification will be described first. In the first modification, dynamic focusing in which a reception focusing point is continuously moved in the direction of depth is carried out; therefore, it may be made possible to adjust a delay time by changing a time for which the write switches SWw and the read switches SWr are on. Specific description will be given with reference to FIG. 8. As an example, it will be assumed that the delay time D1 is the time from the center of the integration period (where write switch SWw is on) to when the read switch SWr transitions from off to on. The delay can be shortened from D1 to D2 by lengthening the on time of the write switch SWw from T1 to T2. In this case, D2<D1. Similarly, the delay time can be increased by decreasing the on time of the write switch SWw (not shown). As a result, the reception focusing point can be changed. Incidentally, either write switch SWw or read switch SWr may be adjustable.

Incidentally, the time for which the write switches SWw and the read switches SWr are on is adjusted based on a signal from a control unit, not shown, provided by the apparatus main unit 101 or a controller in the probe.

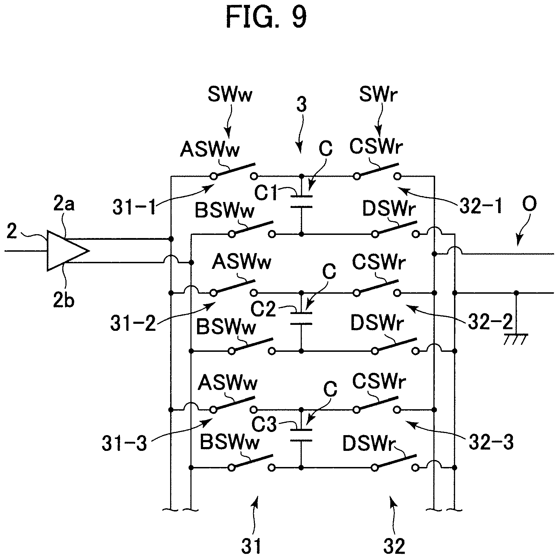

Description will be given to a second modification. The above-mentioned delay unit 3 may be configured as illustrated in FIG. 9. More specific description will be given. In this example, each of the write switches SWw is-includes a first switch ASWw and a second switch BSWw that are synchronously turned on and off and each of the read switches SWr includes a third switch CSWr and a fourth switch DSWr that are synchronously turned on and off.

One end of each first switch ASWw is connected with an output-side first terminal 2a of an amplification unit 2 mentioned above and the other end thereof is connected with one end of a capacitor C mentioned above. One end of each corresponding second switch BSWw is connected with the other end of the capacitor C and the other end thereof is connected with an output-side second terminal 2b of the amplification unit 2.

One end of the corresponding third switch CSWr is connected with the one end of the capacitor C and the other end thereof is connected with the output line O mentioned above. One end of the corresponding fourth switch DSWr is connected with the other end of the capacitor C and the other end thereof is connected with ground.

The loop including the first switch ASWw, the capacitor C, and the second switch BSWw further includes a write circuit 31 that integrates the output current from the corresponding amplification unit 2 to the capacitor C. The loop including the third switch CSWr, the capacitor C, and the fourth switch DSWr θ further includes a read circuit 32 that reads the charge written to the capacitor C. Also in the second modification, multiple write circuits 31 and multiple read circuits 32 mentioned above are provided in parallel to one another (write circuits 31-1 ,31-2, 31-3, ..., read circuits 32-1, 32-2, 32-3, ...).

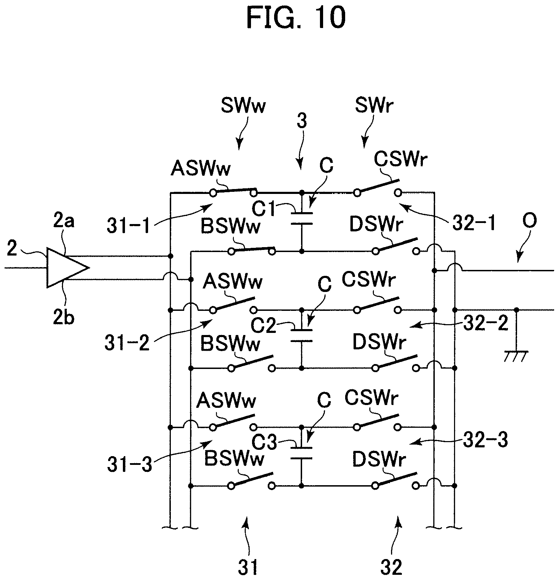

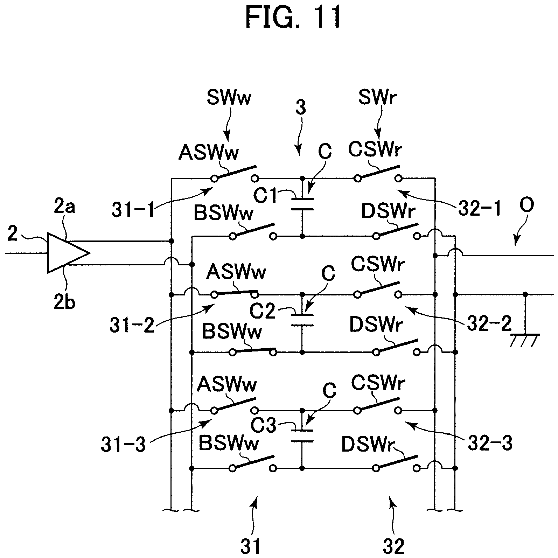

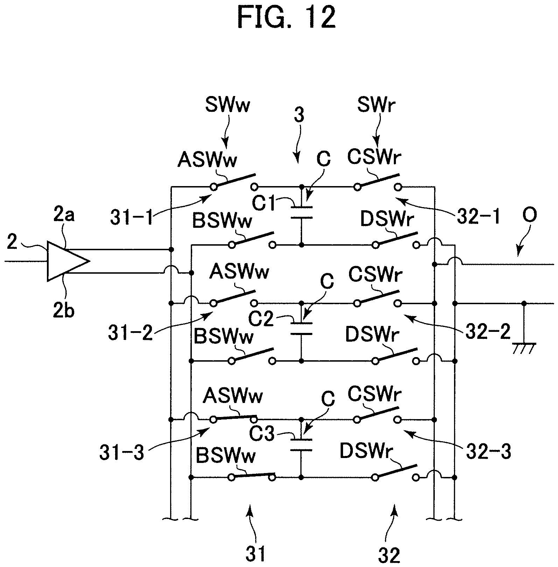

Description will be given to the operation of each the write circuit 31 and each the read circuit 32 in the second modification. When the output current of an amplification unit 2 mentioned above is integrated onto a capacitor C mentioned above, for example, when the write circuit 31-1 integrates the current to the capacitor C1, the following takes place as illustrated in FIG. 10: the first switch ASWw and second switch BSWw of the write circuit 31-1 are synchronously turned on. After first switch ASWw and second switch BSWw of the write circuit 31-1 turn off, the first switch ASWw and second switch BSWw of another individual write circuit among the write circuits 31 are synchronously turned on so that the current is sequentially written to the capacitors C in any write circuit 31. Specifically, next to the write circuit 31-1, the first switch ASWw and second switch BSWw of the write circuit 31-2 are synchronously turned on as illustrated in FIG. 11; and next to the write circuit 31-2, the first switch ASWw and second switch BSWw of the write circuit 31-3 are synchronously turned on as illustrated in FIG. 12. Also in this example, as a result, each amplification unit 2 is constantly connected with one of the capacitors C1-Cn.

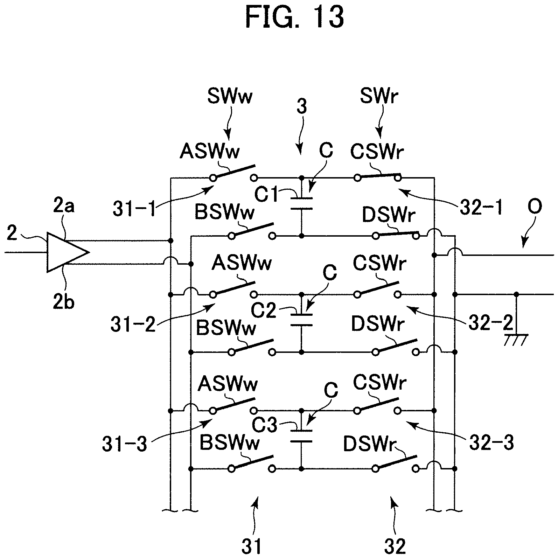

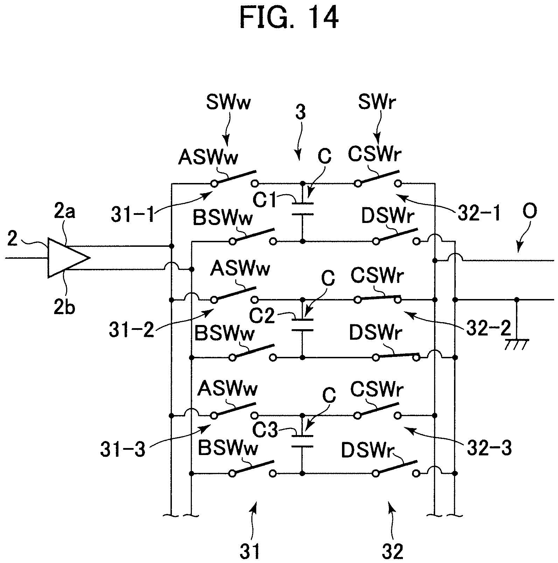

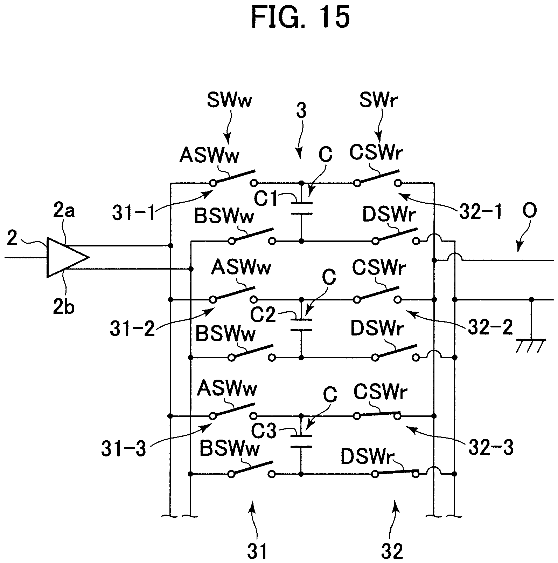

Description will be given to cases where the current written to a capacitor C mentioned above is read. For example, when the read circuit 32-1 reads the current of the capacitor C, the third switch CSWr and fourth switch DSWr of the read circuit 32-1 are synchronously turned on as illustrated in FIG. 13. After third switch CSWr and forth switch DSWr of the read circuit 32-1 turn off, the third switches CSWr and fourth switches DSWr in another individual read circuit of the read circuits 32 are sequentially turned on so that the current of another capacitor C is sequentially read in one of the read circuits 32. Specifically, next to the read circuit 32-1, the third switch CSWr and fourth switch DSWr of the read circuit 32-2 are synchronously turned on as illustrated in FIG. 14; and next to the read circuit 32-2, the third switch CSWr and fourth switch DSWr of the read circuit 32-3 are synchronously turned on as illustrated in FIG. 15.

Incidentally, the delay time D is a time from when each the first switch ASWw and the corresponding second switch BSWw transition from on to off to when the following takes place: the third switch CSWr and the fourth switch DSWr that read the current written by the first switch ASWw and the second switch BSWw are turned on.

### Second Embodiment

Description will be given to a second embodiment. The same elements as in the first embodiment will be marked with the same reference numerals or codes and the description thereof will be omitted.

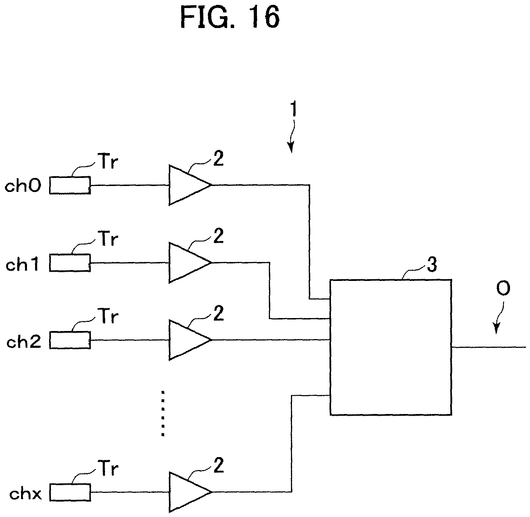

In the first embodiment, the delay unit 3 is provided for the ultrasonic transducer Tr and the amplification unit 2 in each channel. In the second embodiment, meanwhile, the delay unit 3 is provided in common to the ultrasonic transducers Tr and amplification units 2 in multiple channels. In this example, as illustrated in FIG. 16, one delay unit 3 is provided in common to the ultrasonic transducers Tr and the amplification units 2 in multiple channels. An echo signal received at the ultrasonic transducer Tr in each channel is amplified at the corresponding amplification unit 2 and is inputted as a current to the delay unit 3 without exception.

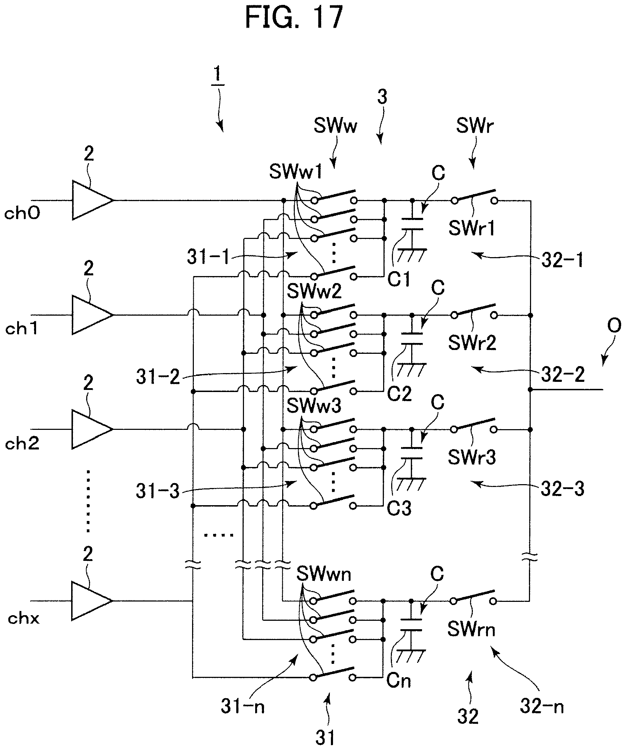

Description will be given to the configuration of the delay unit 3 in this example with reference to FIG. 17. It will be assumed that the number of channels of ultrasonic transducers Tr is (x+1). In this example, (x+1) sets of write switches [SWw1, SWw2, SWw3, ..., SWwn] of the write circuits 31-1, 31-2, 31-3, ..., 31-n are provided in parallel to one another. The (x+1) times (n) write switches are connected with the amplification units 2 in the individual channels.

Also in this example, the write switches SWw and the read switches SWr are turned on and off so that echo signals in each channel are delayed by a predetermined delay time D. Also in this example, the amplification unit 2 in each channel is always connected with one of the capacitors C. In each write circuit 31, incidentally, the multiple write switches SWw may be simultaneously on. In this case, the output currents from the amplification units 2 in multiple channels are added and integrated onto a capacitor C. Therefore, the output currents from the amplification units 2 are added at the delay unit 3.

Also according to the example described up to this point, the same advantages as according to the first embodiment can be obtained. In addition, the risk of saturation in each the capacitor C due to noise can be reduced by sharing the capacitors C among channels.

### Third Embodiment

Description will be given to a third embodiment. The same elements as in the above embodiments will be marked with the same reference numerals and code and the description thereof will be omitted.

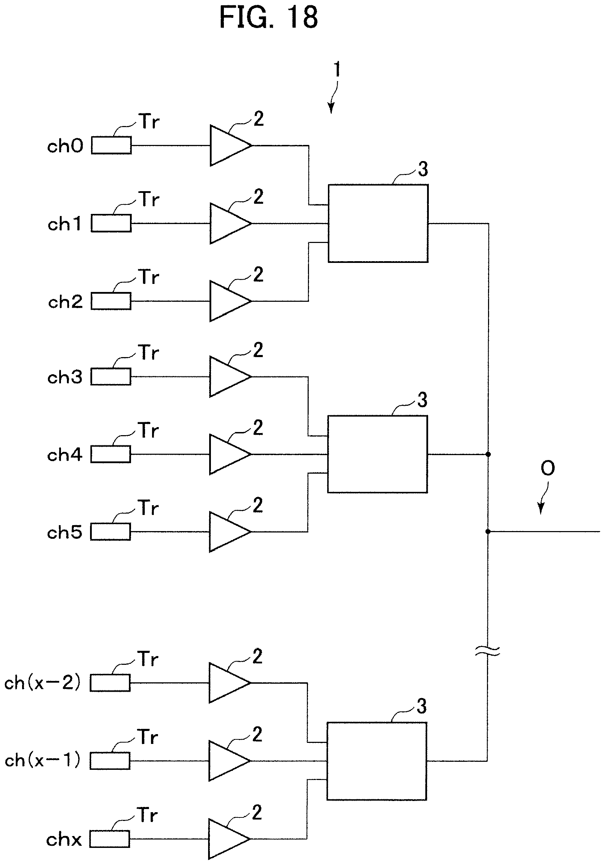

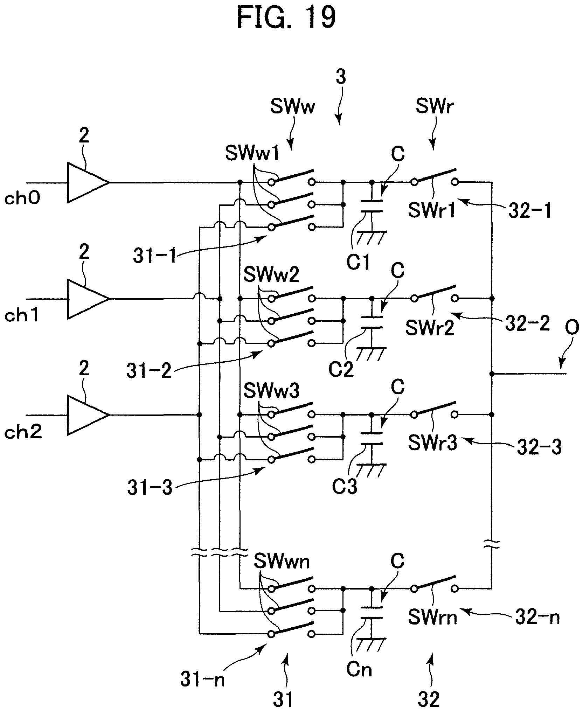

Also in the third embodiment, the delay unit 3 is provided in common to ultrasonic transducers Tr and amplification units 2 in multiple channels as in the second embodiment. Unlike the second embodiment, however, multiple delay units are provided in common to ultrasonic transducers Tr and amplification units 2 in some channels of all the channels. As a result, signals in some channels of a larger group of channels are inputted to each delay unit 3. In this example, one delay unit 3 is provided for ultrasonic transducers Tr and amplification units 2 equivalent to three channels as illustrated in FIG. 18. As illustrated in FIG. 19, therefore, each delay unit 3 is provided with write switches SWw1, SWw2, SWw3, ..., SWwn of the write circuits 31-1, 31-2, 31-3, ..., 31-n on a three-by-three basis. They are connected with the amplification units 2 in the channels with which the delay unit 3 is connected. Incidentally, FIG. 19 depicts the delay unit 3 for ch0 to ch2.

Also according to the example described up to this point, the same advantages as according to the first or second embodiment can be obtained.

Up to this point, description has been given to the invention based on the above embodiments. However, the invention may be variously modified without departing from the subject matter thereof. For example, also in the second and third embodiments, the active current output circuit 4 or a capacitor may be provided in the output line O. Also in the second and third embodiments, the following measure may be taken as in the second modification to the first embodiment: each write switch SWw includes a first switch ASWw and a second switch BSWw provided on a channel-by-channel basis; and each read switch SWr includes a third switch CSWr and a fourth switch DSWr provided on a channel-by-channel basis.

Various aspects and embodiments of the present invention are defined by the following numbered clauses:
1. A receiving circuit comprising:
   an amplification unit amplifying an echo signal received at an ultrasonic transducer for receiving ultrasonic waves; and
   a delay unit providing a delay time to output signals of the amplification unit, wherein the receiving circuit is provided in an ultrasonic probe including the ultrasonic transducer and the amplification unit is comprised of a current output amplifier.
2. The receiving circuit according to clause 1, wherein the delay unit includes:
   a capacitor onto which the output current of the current output amplifier is integrated;
   a write switch writing the output current to the capacitor; and
   a read switch reading the charge from the capacitor.
3. The receiving circuit according to any preceding clause, wherein the capacitor is charged with the output current while the write switch is on.
4. The receiving circuit according to any preceding clause, wherein the delay unit includes a plurality of the capacitors and a plurality of the write switches and the read switches, and the capacitors, the write switches, and the read switches form parallel circuits.
5. The receiving circuit according to any preceding clause,
   wherein when any one of the write switches is turned on, the others are turned off, and
   wherein the current output amplifier is connected with any of the capacitors through the write switch in on state.
6. The receiving circuit according to any preceding clause, wherein the on time of the write switch and/or the read switch is adjustable.
7. The receiving circuit according to any preceding clause, wherein the delay time is a time from when the write switch is turned off to when the read switch is turned on.
8. The receiving circuit according to any preceding clause, wherein the capacitance of the capacitor is smaller than the capacitance of a cable connecting together the ultrasonic probe and the apparatus main unit of an ultrasonic image displaying apparatus.
9. The receiving circuit according to any preceding clause, wherein an active charge amplifier circuit is provided in the stage subsequent to the read switch.
10. The receiving circuit according to any preceding clause, wherein one end of the write switch is connected with the current output amplifier and the other end thereof is connected with one end of the capacitor, the other end of the capacitor is connected with ground, and one end of the read switch is connected with the one end of the capacitor and the other end thereof is connected with an output line.
11. The receiving circuit according to any preceding clause,
   wherein the write switch is comprised of a first switch and a second switch synchronously turned on and off,
   wherein the read switch is comprised of a third switch and a fourth switch synchronously turned on and off, and
   wherein one end of the first switch is connected with an output-side first terminal of the current output amplifier and the other end thereof is connected with the one end of the capacitor, one end of the second switch is connected with the other end of the capacitor and the other end thereof is connected with an output-side second terminal of the current output amplifier, one end of the third switch is connected with the one end of the capacitor and the other end thereof is connected with the output line, and one end of the fourth switch is connected with the other end of the capacitor and the other end thereof is connected with ground.
12. The receiving circuit according to any preceding clause, wherein output currents of the delay unit are added at an output line from the delay unit.
13. The receiving circuit according to any preceding clause, wherein the delay unit is provided for each of the ultrasonic transducers.
14. The receiving circuit according to any preceding clause, wherein the delay unit is provided in common to ultrasonic transducers in a plurality of channels.
15. The receiving circuit according to any preceding clause, wherein the delay unit is singularly provided in common to ultrasonic transducers in all the channels.
16. The receiving circuit according to any preceding clause, wherein a plurality of the delay units are provided in common to ultrasonic transducers in some channels of all the channels.
17. The receiving circuit according to any preceding clause, wherein the output currents from multiple amplification units are added in the delay unit or an output line from the delay unit.
18. The receiving circuit according to any preceding clause, wherein the current output amplifier is either a V/I amplifier amplifying input signals as voltage signals and converting the input signals into current signals and outputting the current signals or an I/I amplifier amplifying input signals as current signals and outputting current signals.
19. An ultrasonic probe provided with the receiving circuit according to any preceding clause.
20. An ultrasonic image displaying apparatus equipped with the ultrasonic probe according to any preceding clause.

## Claims

1. A receiving circuit (1) comprising:
an amplification unit (2) for amplifying an echo signal received at an ultrasonic transducer (102) for receiving ultrasonic waves; and
a delay unit (3) for providing a delay time to output signals of the amplification unit (2), wherein the receiving circuit is provided in an ultrasonic probe including the ultrasonic transducer and the amplification unit (2) is comprised of a current output amplifier.

2. The receiving circuit (1) according to claim 1, wherein the delay unit (3) includes:
a capacitor onto which the output current of the current output amplifier is integrated;
a write switch writing the output current to the capacitor; and
a read switch reading the charge from the capacitor.

3. The receiving (1) circuit according to any preceding claim, wherein the capacitor is charged with the output current while the write switch is on.

4. The receiving circuit (1) according to any preceding claim, wherein the delay unit (3) includes a plurality of the capacitors and a plurality of the write switches and the read switches, and the capacitors, the write switches, and the read switches form parallel circuits.

5. The receiving circuit (1) according to any preceding claim,
wherein when any one of the write switches is turned on, the others are turned off, and
wherein the current output amplifier is connected with any of the capacitors through the write switch in on state.

6. The receiving circuit (1) according to any preceding claim, wherein the on time of the write switch and/or the read switch is adjustable.

7. The receiving circuit (1) according to any preceding claim, wherein the delay time is a time from when the write switch is turned off to when the read switch is turned on.

8. The receiving circuit (1) according to any preceding claim, wherein the capacitance of the capacitor is smaller than the capacitance of a cable connecting together the ultrasonic probe and the apparatus main unit of an ultrasonic image displaying apparatus (104).

9. The receiving circuit (1) according to any preceding claim, wherein an active charge amplifier circuit is provided in the stage subsequent to the read switch.

10. The receiving circuit (1) according to any preceding claim, wherein one end of the write switch is connected with the current output amplifier and the other end thereof is connected with one end of the capacitor, the other end of the capacitor is connected with ground, and one end of the read switch is connected with the one end of the capacitor and the other end thereof is connected with an output line.

11. The receiving circuit (1) according to any preceding claim,
wherein the write switch is comprised of a first switch and a second switch synchronously turned on and off,
wherein the read switch is comprised of a third switch and a fourth switch synchronously turned on and off, and
wherein one end of the first switch is connected with an output-side first terminal of the current output amplifier and the other end thereof is connected with the one end of the capacitor, one end of the second switch is connected with the other end of the capacitor and the other end thereof is connected with an output-side second terminal of the current output amplifier, one end of the third switch is connected with the one end of the capacitor and the other end thereof is connected with the output line, and one end of the fourth switch is connected with the other end of the capacitor and the other end thereof is connected with ground.

12. The receiving circuit (1) according to any preceding claim, wherein output currents of the delay unit (3) are added at an output line from the delay unit.

13. The receiving circuit (1) according to any preceding claim, wherein the delay unit (3) is provided for each of the ultrasonic transducers.

14. The receiving circuit (1) according to any preceding claim, wherein the delay unit (3) is provided in common to ultrasonic transducers in a plurality of channels.

15. The receiving circuit (1) according to any preceding claim, wherein the current output amplifier is either a V/I amplifier amplifying input signals as voltage signals and converting the input signals into current signals and outputting the current signals or an I/I amplifier amplifying input signals as current signals and outputting current signals.
